# EUROPEAN PATENT SPECIFICATION

(11) **EP 4 104 764 B1**
(45) Date of publication and mention of the grant of the patent: **15.05.2024**
(21) Application number: 22178855.7
(22) Date of filing: 14.06.2022
(51) Int. Cl.: A61B 5/367, A61B 5/00

(54) **IMPROVING VISUALIZATION OF ELECTRICAL SIGNALS PROPAGATING OVER THE SURFACE OF PATIENT ORGAN**
VERBESSERUNG DER VISUALISIERUNG VON ELEKTRISCHEN SIGNALEN, DIE SICH ÜBER DIE OBERFLÄCHE EINES ORGANS EINES PATIENTEN AUSBREITEN
AMÉLIORATION DE LA VISUALISATION DE SIGNAUX ÉLECTRIQUES SE PROPAGEANT SUR LA SURFACE D'UN ORGANE DE PATIENT

(30) Priority: 15.06.2021 US 202117348002
(43) Date of publication of application: 21.12.2022
(73) Proprietor: Biosense Webster (Israel) Ltd, Yokneam, 2066717 (IL)
(72) Inventor: GOVARI, Assaf, 2066717 Yokneam (IL); ALTMANN, Andres Claudio, 2066717 Yokneam (IL); GLINER, Vadim, 2066717 Yokneam (IL); PALTI, Yair, 2066717 Yokneam (IL)
(74) Representative: Carpmaels & Ransford LLP

(56) References cited:
- US-A1- 2018 325 400
- US-A1- 2020 146 572
- US-A1- 2021 000 369
- MASE M ET AL: "Automatic reconstruction of activation and velocity maps from electro-anatomic data by radial basis functions", 2010 ANNUAL INTERNATIONAL CONFERENCE OF THE IEEE ENGINEERING IN MEDICINE AND BIOLOGY SOCIETY : (EMBC 2010) ; BUENOS AIRES, ARGENTINA, 31 AUGUST - 4 SEPTEMBER 2010, IEEE, PISCATAWAY, NJ, USA, 31 August 2010 (2010-08-31), pages 2608-2611, XP032108610, DOI: 10.1109/IEMBS.2010.5626616 ISBN: 978-1-4244-4123-5

## Description

### FIELD OF THE INVENTION

The present invention relates generally to medical devices, and particularly to methods and systems for presenting two-dimensional projection of wave vectors over a three-dimensional map of an organ.

### BACKGROUND OF THE INVENTION

Various techniques for visualizing propagation of electrical signals over a surface of an organ have been published.

For example, U.S. Patent Application Publication 2018/0325400 describes systems and methods to determine conduction velocity and to generate one or more conduction velocity and/or pattern maps to facilitate identification of arrhythmogenic mechanisms. The method includes defining a region of interest around a given node on a three-dimensional surface of interest, e.g. a cardiac envelope. Electrophysiological signals are stored, in memory, for each of a plurality of nodes distributed across the surface of interest. The method also includes orthogonally projecting the region of interest for the given node from the surface of interest onto a two-dimensional surface tangential to the given node. The method also includes estimating a two-dimensional conduction velocity vector for the given node based on analysis of the electrophysiological signal for the given node relative to other nodes in the region of interest. The method also includes estimating a three-dimensional conduction velocity vector for the given node based on the two-dimensional conduction velocity vector.

U.S. Patent Application Publication 2020/0375489 describes a method including receiving, in a processor, a two-dimensional (2D) electroanatomical (EA) map of an interior surface of at least a portion of a cavity of an organ of a patient, the 2D EA map including electrophysiological (EP) values measured at respective locations on the interior surface. A complex analytic function is fitted to a set of the EP values that were measured in a given region of the 2D EA map, and a singularity is identified in the fitted complex analytic function.

### SUMMARY OF THE INVENTION

An embodiment of the present invention that is described herein provides a system including a display and a processor. The processor is configured to: (i) receive a three-dimensional (3D) anatomical map including multiple vectors, and a selection of a section of the 3D anatomical map, the section including one or more given vectors among the multiple vectors, (ii) produce a two-dimensional (2D) projection of the section, which includes the one or more given vectors, and (iii) present on the display, the 2D projection overlaid on the 3D anatomical map.

In some embodiments, the 3D anatomical map includes a map of at least a portion of a heart, and the multiple vectors include wave vectors indicative of electrical signals propagating over a surface of the heart. In other embodiments, the 3D anatomical map has a first magnification, and the processor is configured to present the 2D projection at a second magnification, different from the first magnification.

In an embodiment, the processor is configured to present the 2D projection overlaid on the 3D anatomical map at a position of the section. In another embodiment, the 2D projection includes the one or more given vectors and a background, which includes the 2D projection of one or more anatomical features of the section of the 3D anatomical map, and the processor is configured to alter the background, so as increase a contrast between the wave vectors and the background.

There is additionally provided, in accordance with an embodiment of the present invention, a processor-implemented method including receiving at a processor (i) a three-dimensional (3D) anatomical map including multiple vectors, and (ii) a selection of a section of the 3D anatomical map, the section including one or more given vectors among the multiple vectors. A two-dimensional (2D) projection of the section, which includes the one or more given vectors, is produced. The 2D projection is presented overlaid on the 3D anatomical map.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a catheter-based tracking and ablation system, in accordance with an embodiment of the present invention;
Fig. 2A is a schematic, pictorial illustration of a three-dimensional (3D) anatomical map, in accordance with an embodiment of the present invention;
Fig. 2B is a schematic, pictorial illustration of a a two-dimensional (2D) projection of a section of the 3D anatomical map, which is overlaid on the 3D anatomical map, in accordance with an embodiment of the present invention; and
Fig. 3 is a flow charts that schematically illustrate a method for visualizing wave vectors using a 2D projection of a section, overlaid on a 3D anatomical map, in accordance with embodiments of the present invention.

### DETAILED DESCRIPTION OF EMBODIMENTS

### OVERVIEW

Some medical procedures, such as cardiac radiofrequency (RF) ablation, require electro-anatomical mapping of the heart before performing the ablation. In some cases the mapping requires visualization of electrical signals propagating over a surface of the heart. Due to the three-dimensional (3D) nature of the heart, and the complexity of vectors associated with the propagating signals, it is difficult to obtain a clear visualization of the vectors over the 3D map.

Embodiments of the present invention that are described hereinbelow provide improved techniques for visualizing wave vectors presented over a 3D map of a patient heart.

In some embodiments, a system for visualizing wave vectors and presenting the wave vectors over a heart map comprises a display and a processor.

In some embodiments, the processor is configured to receive a 3D anatomical map of at least a portion of the patient heart, also referred to herein as a map, for brevity. The map comprising multiple wave vectors, also referred to herein as vectors, which are indicative of electrical signals propagating over the surface of the patient heart.

In some embodiments, the processor is configured to receive, e.g., from a user, a selection of a section of the 3D anatomical map. The selected section comprises one or more vectors from among the multiple vectors of the originally-received 3D anatomical map. The vectors within the selected section are also referred to herein as given vectors.

In some embodiments, the processor is configured to produce a two-dimensional (2D) projection of the section, which comprises the one or more given vectors. Moreover, the processor is configured to present on the display, the 2D projection of the section overlaid on the 3D anatomical map.

In some embodiments, the processor is configured to present the 2D projection over the map at the original position of the selected section. Moreover, the processor is configured to present the 2D projection at a different magnification compared to that of the 3D anatomical map. In the present example, the magnification of the 2D projection is larger than that of the 3D anatomical map, so as to improve the visualization of the wave vectors.

In some cases, the background of the 3D anatomical map comprises one or more anatomical features and various sorts of annotations that are presented over the map. The anatomical features and the annotations that appear in the background, may produce noise that may interfere with the presentation of the 2D-projected wave vectors. In some embodiments, the processor is configured to alter the background using any suitable technique, so as to reduce the noise-level caused by the background and increase the contrast between the wave vectors and the background. For example, the processor is configured to blur the background, so as to improve the contrast between the presented wave vectors and the background. Note that the projection and presentation techniques described above, may be used for visualizing any signal propagating over a surface, or within a volume, of any organ in a patient body.

The disclosed techniques provide improved visualization and presentation of signals propagating over a surface of a patient organ. More particularly, the disclosed techniques improve the contrast of the presentation by (i) presenting, over a 3D map of the organ, a 2D projection of vectors indicative of the propagated signal, and (ii) reducing the noise-level caused by the 3D map of the organ in question.

### SYSTEM DESCRIPTION

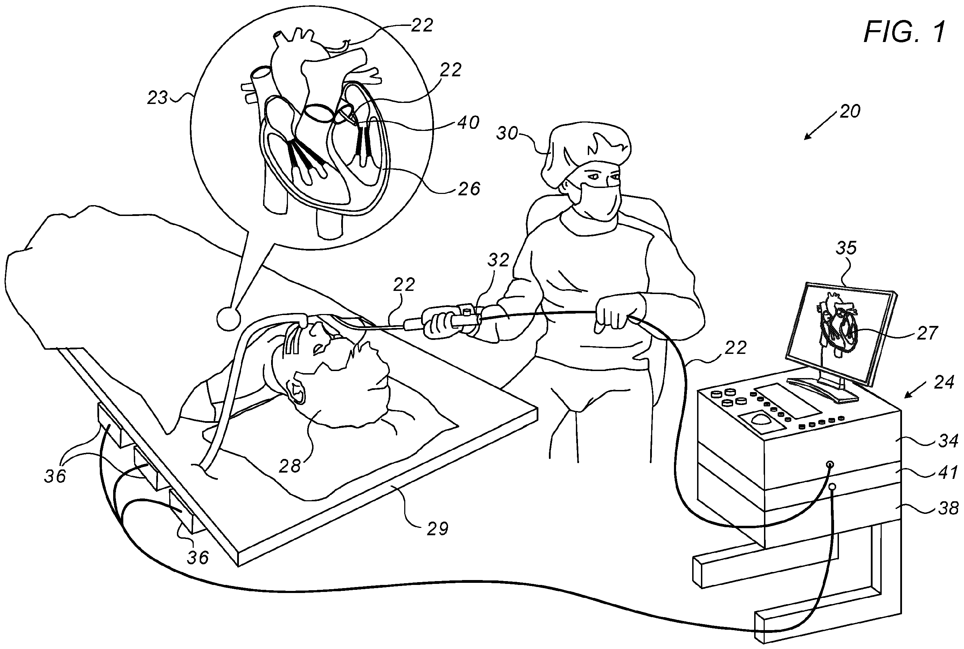

Fig. 1 is a schematic, pictorial illustration of a catheter-based tracking and ablation system 20, in accordance with an embodiment of the present invention.

In some embodiments, system 20 comprises a catheter 22, in the present example a cardiac catheter, and a control console 24. In the embodiment described herein, catheter 22 may be used for any suitable therapeutic and/or diagnostic purposes, such as ablation of tissue in a heart 26.

In some embodiments, console 24 comprises a processor 34, typically a general-purpose computer, with suitable front end and interface circuits 38 for receiving signals via catheter 22 and for controlling the other components of system 20 described herein. Console 24 further comprises a user display 35, which is configured to receive from processor 34 a map 27 of heart 26, and to display map 27.

In some embodiments, map 27 may comprise any suitable type of three-dimensional (3D) anatomical map produced using any suitable technique. For example, the anatomical map may be produced using an anatomical image produced by using a suitable medical imaging system, or using a fast anatomical mapping (FAM) techniques using the CARTO^{™} system, produced by Biosense Webster Inc. (Irvine, Calif.), or using any other suitable technique, or using any suitable combination of the above.

Reference is now made to an inset 23. In some embodiments, prior to performing an ablation procedure, a physician 30 inserts catheter 22 through the vasculature system of a patient 28 lying on a table 29, so as to perform electro-anatomical mapping of tissue in question of heart 26.

In some embodiments, catheter 22 comprises a distal-end assembly 40 having multiple sensing electrodes (not shown). For example, distal-end assembly 40 may comprise: (i) a basket catheter having multiple splines, each spline having multiple sensing electrodes, (ii) a balloon catheter having multiple sensing electrodes disposed on the surface of the balloon, or (iii) a focal catheter having multiple sensing electrodes. Each sensing electrode is configured to produce, in response to sensing electrophysiological (EP) signals in tissue of heart 26, one or more signals indicative of the sensed EP signals.

In some embodiments, the proximal end of catheter 22 is connected, *inter alia,* to interface circuits 38, so as to transfer these signals to processor 34 for performing the electro-anatomical mapping.

In some embodiments, during the electro-anatomical mapping, the signals produced by the sensing electrodes of distal-end assembly 40 may comprise thousands of data points, e.g., about 50,000 data points or even more. Based on the data points, processor 34 is configured to present on map 27, vectors indicative of electrical signals propagating over the surface of heart 26, and based on the presented vectors, physician 30 determines one or more sites for ablating tissue in heart 26. However, physician 30 may have difficulties to review and analyze the aforementioned large number of data points and vectors, which may prolong the duration of the ablation procedure. Moreover, the large amount of data points and vectors may confuse physician 30, and therefore, may reduce the quality of the ablation procedure.

In the context of the present disclosure and in the claims, the terms "about" or "approximately" for any numerical values or ranges indicate a suitable dimensional tolerance that allows the part or collection of components to function for its intended purpose as described herein.

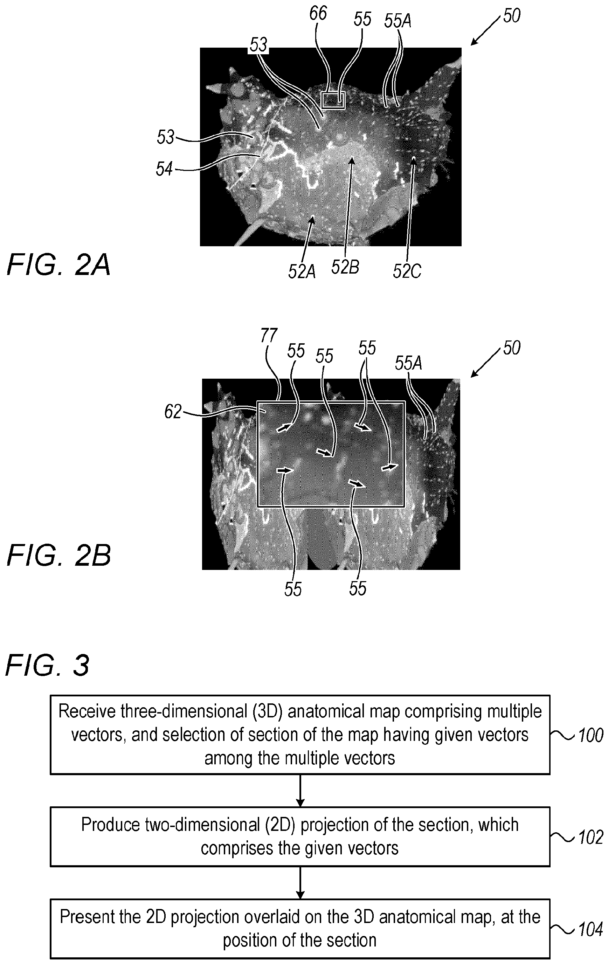

In some embodiments, processor 34 is configured to present the vectors, e.g., on display 35, in two visualization layers: (i) a 3D presentation of the vectors (also referred to herein as wave vectors, and (ii) in response to selection of a section of interest in map 27 by physician 30 (or by any other user of system 20), display a two-dimensional (2D) projection of the section of interest overlaid on the 3D anatomical map. The visualization and presentation is described in detail in Figs. 2A and 2B below.

In other embodiments, catheter 22 may comprise one or more ablation electrodes (not shown) coupled to distal-end assembly 40. The ablation electrodes are configured to ablate tissue at a target location of heart 26. After determining the ablation plan, physician 30 navigates distal-end assembly 40 in close proximity to the target location in heart 26 by using a manipulator 32 for manipulating catheter 22. Additionally or alternatively, physician 30 may use any different sort of suitable catheter for ablating tissue of heart 26 so as to carry out the aforementioned ablation plan.

In some embodiments, the position of distal-end assembly 40 in the heart cavity is measured using a position sensor (not shown) of a magnetic position tracking system. In the present example, console 24 comprises a driver circuit 41, which is configured to drive magnetic field generators 36 placed at known positions external to patient 28 lying on table 29, e.g., below the patient's torso. The position sensor is coupled to the distal end, and is configured to generate position signals in response to sensed external magnetic fields from field generators 36. The position signals are indicative of the position the distal end of catheter 22 in the coordinate system of the position tracking system.

This method of position sensing is implemented in various medical applications, for example, in the CARTO^{™} system, produced by Biosense Webster Inc. (Irvine, Calif.) and is described in detail in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1.

In some embodiments, the coordinate system of the position tracking system are registered with the coordinate systems of system 20 and map 27, so that processor 34 is configured to display on map 27, the position of the distal end of catheter 22.

In some embodiments, processor 34, typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

This particular configuration of system 20 is shown by way of example, in order to illustrate certain problems that are addressed by embodiments of the present invention and to demonstrate the application of these embodiments in enhancing the performance of such a system. Embodiments of the present invention, however, are by no means limited to this specific sort of example system, and the principles described herein may similarly be applied to other sorts of medical systems.

Fig. 2A is a schematic, pictorial illustration of a 3D anatomical map 50, in accordance with an embodiment of the present invention. Anatomical map 50, which is also referred to herein as a map 50, for brevity, comprises a map of at least a portion of heart 26, and may replace, for example, map 27 of Fig. 1 above.

In some embodiments, map 50 has anatomical features of heart 26, and comprises various types of display objects, which are overlaid on the anatomical features. The displayed objects are indicative of measurements carried out during the electro-anatomical mapping described in Fig. 1 above. The display objects are indicative of parameters obtained based on the signals produced by the sensing electrodes of distal-end assembly 40.

In the present example, the display objects comprise: (i) different colors 52A, 52B and 52C indicative of the voltage measured at respective locations on tissue of heart 26, (ii) clusters 53, each of which indicative of data points having common measured and/or calculated properties of heart 26, (iii) an ablation line 54, and (iv) vectors 55 and 55A, also referred to herein as wave vectors 55 and 55A, respectively, which are indicative of the speed and direction of electrical signals propagating over the surface of heart 26.

In some embodiments, physician 30 may use an input device (e.g., a mouse, a track ball, or a touch screen) of console 24 to select a section 66 indicative of a region of interest (ROI) within map 50. Note that vectors 55 and 55A comprise similar wave vectors, and receive different numerals for the sake of presentation and conceptual clarity. The different numerals (55 and 55A) are indicative of the different respective positions of the wave vectors in map 50. In the present example, vectors 55 are positioned within section 66 and vectors 55A are positioned out of section 66.

In some embodiments, physician 30 may wish to review one or more of vectors 55 of section 66, which are among the wave vectors (i.e., vectors 55A) of map 50. In some cases, vectors 55 may appear small and not sufficiently clear in the 3D display of section 66 as shown in Fig. 2A. Thus, physician 30 may need an improved the display of vectors 55 in order to be able to analyze the electrical signals propagating, within section 66, over the surface of heart 26.

In other embodiment, due to the excess of data presented over the 3D anatomical map, processor 34 may not present any wave vectors over map 50 of Fig. 2A. In such embodiments, physician 30 may require a local display of vectors 55 within section 66.

### PROJECTION OF 2D WAVE VECTORS OVER A 3D HEART MAP

Fig. 2B is a schematic, pictorial illustration of a 2D projection 77 of section 66, overlaid on map 50, in accordance with an embodiment of the present invention.

In some embodiments, processor 34 is configured to produce 2D projection 77 of section 66, which comprises vectors 55 as described in Fig. 2A above. In the present example, processor 34 is configured to present vectors 55 emphasized, and to alter, e.g., blur, a background 62 of section 66, so as to improve the contrast between vectors 55 and background. In the context of the present disclosure and in the claims, the term "background" refers to all the elements and/or components that appear within section 66, except for vectors 55.

In some embodiments, by altering (e.g., blurring) background 62 and emphasizing vectors 55 within 2D projection 77, processor 34 provides physician 30 with a clear presentation and indication of the direction, and optionally the speed, of the aforementioned electrical signals propagating within section 66, over the surface of heart 26.

In some embodiments, processor 34 is configured to produce a two-level visualization of the vectors in map 50. For example, vectors 55 that are located within 2D projection 77 of section 66, are displayed using a magnification higher than that of vectors 55A located out of section 66. In other words, 2D projection 77 serves as a "magnifying and emphasizing glass" of the wave vectors positioned within the ROI of map 50. Note that the magnification and emphasizing is carried out in response to a selection of section 66 by physician 30 or any other user of system 20.

In other embodiments, 2D projection 77 of Fig. 2B, and section 66 shown in Fig. 2A may have a similar magnification, or any other different magnification from one another.

In other embodiments, instead of blurring background 62, processor 34 is configured to emphasize vectors 55 using any other suitable technique, such as but not limited to, elimination of background 62, or by displaying background 62 using a flat color, and vectors 55 using a different color having high contract to the color selected for the background.

In alternative embodiments, processor 34 is configured to display vectors 55 emphasized over the original background of map 50, e.g., the background of section 66 of Fig. 2A above, which is presented using the same magnification of 2D projection 77.

In some embodiments, processor 34 is configured to present 2D projection 77 overlaid on map 50 at the original position of section 66. In the present example, 2D projection 77 has higher magnification compared to that of section 66. In such embodiments, processor 34 is configured to display, on display 35, the center-of-gravity of 2D projection 77 at the original position of the center-of-gravity of section 66.

In other embodiments, processor 34 is configured to present 2D projection 77 over any other suitable position of map 50.

In other embodiment, due to the excess of data presented over the 3D anatomical map, processor 34 may omit vectors 55A from map 50 (for reducing the amount of details within map 50). In such embodiments, processor 34 is configured to present over map 50, only vectors 55, which are the wave vectors positioned within the ROI (e.g., section 66) of map 50.

The configuration of maps 50 presented in Figs. 2A and 2B is shown by way of example. In other embodiments, maps 50 may comprise any other suitable configuration of display objects presented for visualizing any feature of interest measured within at least a portion of heart 26.

Fig. 3 is a flow charts that schematically illustrate a method for visualizing wave vectors 55 using 2D projection 77 of section 66 presented over 3D anatomical map 50, in accordance with embodiments of the present invention.

The method begins at an information receiving step 100, with processor 34 receiving 3D anatomical map 50 comprising multiple vectors 55 and 55A, and a selection (e.g., by physician 30) of section 66 of the map having vectors 55 among the multiple vectors 55 and 55A of map 50, as described in detail in Figs. 2A and 2B above.

At a 2D projection step 102, processor 34 produces 2D projection 77 of section 66. As described in detail in Fig. 2B above, 2D projection 77 comprises vectors 55, also referred to herein as given vectors.

At a presentation step 104 that concludes the method, processor 34 presents 2D projection 77 overlaid on map 50, at the original position of section 66, which is shown in Fig. 2A above.

In some embodiments, map 50 comprises a 3D anatomical or electro-anatomical map of at least a portion of heart 26. Moreover, vectors 55 and 55A comprise wave vectors indicative of electrical signals propagating over the surface of heart 26, as described in Figs. 2A and 2B above.

In some embodiments, processor 34 presents 2D projection 77 using a magnification that is different, typically larger, from that of section 66, as shown and described in Fig. 2B above.

In some embodiments, during the presentation of 2D projection 77, processor 34 may blur or eliminate background 62 of 2D projection 77. Moreover, processor 34 may emphasize the appearance of vectors 55, so that physician 30 can have a clear visibility of the direction and the magnitude of the electrical signals propagating over the surface of heart 26 in the area covered by section 66.

Although the embodiments described herein mainly address visualization of electrical signals propagating over a heart map, the methods and systems described herein can also be used in other applications, such as in visualization of any dynamic process carried out within any organ of a patient, such as the patient brain.

It will thus be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art. The scope of the invention is defined by the appended independent claims. Optional features are presented in the appended dependent claims.

## Claims

1. A system, comprising:
a display; and
a processor, which is configured to: (i) receive a three-dimensional (3D) anatomical map comprising multiple vectors, and a selection of a section of the 3D anatomical map, wherein the section comprising one or more given vectors among the multiple vectors, (ii) produce a two-dimensional (2D) projection of the section, which comprises the one or more given vectors, and (iii) present on the display, the 2D projection overlaid on the 3D anatomical map.

2. The system according to claim 1, wherein the 3D anatomical map comprises a map of at least a portion of a heart, and wherein the multiple vectors comprise wave vectors indicative of electrical signals propagating over a surface of the heart.

3. The system according to claim 1, wherein the 3D anatomical map has a first magnification, and wherein the processor is configured to present the 2D projection at a second magnification, different from the first magnification.

4. The system according to claim 1, wherein the processor is configured to present the 2D projection overlaid on the 3D anatomical map at a position of the section.

5. The system according to claim 1, wherein the 2D projection comprises the one or more given vectors and a background, which comprises the 2D projection of one or more anatomical features of the section of the 3D anatomical map, and wherein the processor is configured to alter the background, so as increase a contrast between the wave vectors and the background.

6. A processor-implemented method, comprising:
receiving at a processor (i) a three-dimensional (3D) anatomical map comprising multiple vectors, and (ii) a selection of a section of the 3D anatomical map, wherein the section comprising one or more given vectors among the multiple vectors;
producing a two-dimensional (2D) projection of the section, which comprises the one or more given vectors; and
presenting the 2D projection overlaid on the 3D anatomical map.

7. The method according to claim 6, wherein the 3D anatomical map comprises a map of at least a portion of a heart, and wherein the multiple vectors comprise wave vectors indicative of electrical signals propagating over a surface of the heart.

8. The method according to claim 6, wherein the 3D anatomical map has a first magnification, and wherein producing the 2D projection comprises presenting the 2D projection at a second magnification, different from the first magnification.

9. The method according to claim 6, wherein presenting the 2D projection comprises presenting the 2D projection overlaid on the 3D anatomical map at a position of the section.

10. The method according to claim 6, wherein the 2D projection comprises the one or more given vectors and a background, which comprises the 2D projection of one or more anatomical features of the section of the 3D anatomical map, and wherein producing the 2D projection comprises altering the background, so as increase a contrast between the wave vectors and the background.

## Patentansprüche

1. System, umfassend:
eine Anzeige; und
einen Prozessor, der konfiguriert ist zum: (i) Empfangen einer dreidimensionalen (3D) anatomischen Karte, umfassend mehrere Vektoren, und einer Auswahl eines Abschnitts der anatomischen 3D-Karte, wobei der Abschnitt einen oder mehrere gegebene Vektoren unter den mehreren Vektoren umfasst, (ii) Produzieren einer zweidimensionalen (2D) Projektion des Abschnitts, die den einen oder die mehreren gegebenen Vektoren umfasst, und (iii) Präsentieren, auf der Anzeige, der 2D-Projektion überlagert auf der anatomischen 3D-Karte.

2. System nach Anspruch 1, wobei die anatomische 3D-Karte eine Karte mindestens eines Teils eines Herzens umfasst und wobei die mehreren Vektoren Wellen-Vektoren umfassen, die elektrische Signale angeben, die sich über eine Oberfläche des Herzens ausbreiten.

3. System nach Anspruch 1, wobei die anatomische 3D-Karte eine erste Vergrößerung aufweist und wobei der Prozessor konfiguriert ist zum Präsentieren der 2D-Projektion bei einer zweiten Vergrößerung, die von der ersten Vergrößerung verschieden ist.

4. System nach Anspruch 1, wobei der Prozessor konfiguriert ist zum Präsentieren der 2D-Projektion überlagert auf der anatomischen 3D-Karte bei einer Position des Abschnitts.

5. System nach Anspruch 1, wobei die 2D-Projektion den einen oder die mehreren gegebenen Vektoren und einen Hintergrund, der die 2D-Projektion eines oder mehrerer anatomischer Merkmale des Abschnitts der anatomischen 3D-Karte umfasst, umfasst und wobei der Prozessor konfiguriert ist, den Hintergrund zu ändern, um einen Kontrast zwischen den Wellen-Vektoren und dem Hintergrund zu vergrößern.

6. Prozessorimplementiertes Verfahren, umfassend:
Empfangen, an einem Prozessor, (i) einer dreidimensionalen (3D) anatomischen Karte, umfassend mehrere Vektoren, und (ii) einer Auswahl eines Abschnitts der anatomischen 3D-Karte, wobei der Abschnitt einen oder mehrere gegebene Vektoren unter den mehreren Vektoren umfasst;
Produzieren einer zweidimensionalen (2D) Projektion des Abschnitts, der den einen oder die mehreren gegebenen Vektoren umfasst; und
Präsentieren der 2D-Projektion überlagert auf der anatomischen 3D-Karte.

7. Verfahren nach Anspruch 6, wobei die anatomische 3D-Karte eine Karte mindestens eines Teils eines Herzens umfasst und wobei die mehreren Vektoren Wellen-Vektoren umfassen, die elektrische Signale angeben, die sich über eine Oberfläche des Herzens ausbreiten.

8. Verfahren nach Anspruch 6, wobei die anatomische 3D-Karte eine erste Vergrößerung aufweist und wobei Produzieren der 2D-Projektion umfasst, die 2D-Projektion bei einer zweiten Vergrößerung zu präsentieren, die von der ersten Vergrößerung verschieden ist.

9. Verfahren nach Anspruch 6, wobei Präsentieren der 2D-Projektion umfasst, die 2D-Projektion überlagert auf der anatomischen 3D-Karte bei einer Position des Abschnitts zu präsentieren.

10. Verfahren nach Anspruch 6, wobei die 2D-Projektion den einen oder die mehreren gegebenen Vektoren und einen Hintergrund, der die 2D-Projektion eines oder mehrerer anatomischer Merkmale des Abschnitts der anatomischen 3D-Karte umfasst, umfasst und wobei Produzieren der 2D-Projektion umfasst, den Hintergrund zu ändern, um einen Kontrast zwischen den Wellen-Vektoren und dem Hintergrund zu vergrößern.

## Revendications

1. Système comprenant :
un dispositif d'affichage ; et
un processeur, qui est configuré pour (i) recevoir une carte anatomique tridimensionnelle (3D) comprenant des vecteurs multiples, et une sélection d'une section de la carte anatomique 3D, la section comprenant un ou plusieurs vecteurs donnés parmi les vecteurs multiples, (ii) produire une projection bidimensionnelle (2D) de la section, qui comprend le ou les vecteurs donnés, et (iii) présenter sur le dispositif d'affichage, la projection 2D superposée sur la carte anatomique 3D.

2. Système selon la revendication 1, la carte anatomique 3D comprenant une carte d'au moins une partie du coeur, et les vecteurs multiples comprenant des vecteurs d'ondes indiquant des signaux électriques se propageant sur une surface du coeur.

3. Système selon la revendication 1, la carte anatomique 3D ayant un premier grossissement, et le processeur étant configuré pour présenter la projection 2D à un second grossissement, différent du premier grossissement.

4. Système selon la revendication 1, le processeur étant configuré pour présenter la projection 2D superposée sur la carte anatomique 3D à une position de la section.

5. Système selon la revendication 1, la projection 2D comprenant le ou les vecteurs donnés et un arrière-plan, qui comprend la projection 2D d'une ou plusieurs caractéristiques anatomiques de la section de la carte anatomique 3D, et le processeur étant configuré pour modifier l'arrière-plan, de manière à augmenter un contraste entre les vecteurs d'ondes et l'arrière-plan.

6. Procédé mis en oeuvre par un processeur, comprenant :
la réception par un processeur (i) d'une carte anatomique tridimensionnelle (3D) comprenant des vecteurs multiples, et (ii) la sélection d'une section de la carte anatomique 3D, la section comprenant un ou plusieurs vecteurs donnés parmi les vecteurs multiples ;
la production d'une projection bidimensionnelle (2D) de la section, qui comprend le ou les vecteurs donnés ; et
la présentation de la projection 2D superposée sur la carte anatomique 3D.

7. Procédé selon la revendication 6, la carte anatomique 3D comprenant une carte d'au moins une partie d'un coeur, et les vecteurs multiples comprenant des vecteurs d'ondes indiquant des signaux électriques se propageant sur une surface du coeur.

8. Procédé selon la revendication 6, la carte anatomique 3D ayant un premier grossissement, et la production de la projection 2D comprenant la présentation de la projection 2D à un second grossissement, différent du premier grossissement.

9. Procédé selon la revendication 6, la présentation de la projection 2D comprenant la présentation de la projection 2D superposée sur la carte anatomique 3D à une position de la section.

10. Procédé selon la revendication 6, la projection 2D comprenant le ou les vecteurs donnés et un arrière-plan, qui comprend la projection 2D d'une ou plusieurs caractéristiques anatomiques de la section de la carte anatomique 3D, et la production de la projection 2D comprenant la modification de l'arrière-plan, de manière à augmenter un contraste entre les vecteurs d'ondes et l'arrière-plan.
